(19) Europäisches Patentamt
European Patent Office
Office européen des brevets

(11) **EP 4 113 525 A1**

(12) **EUROPEAN PATENT APPLICATION**

(43) Date of publication:
**04.01.2023 Bulletin 2023/01**

(21) Application number: **22182009.5**

(22) Date of filing: **29.06.2022**

(51) International Patent Classification (IPC):
**G16H 10/60** (2018.01)   **G16H 50/20** (2018.01)
**G16H 50/30** (2018.01)   **G16H 50/70** (2018.01)

(52) Cooperative Patent Classification (CPC):
**G16H 10/60; G16H 50/20; G16H 50/30; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2021 US 202117364252**

(71) Applicant: **Johnson & Johnson Vision Care, Inc. Jacksonville, FL 32256 (US)**

(72) Inventors:
• **SCALES, Charles Webb Jacksonville, 32256 (US)**
• **CHANG, Chin-Wen Raritan, 08869 (US)**
• **HOLY, Chantal Elisabeth New Brunswick, 08901 (US)**
• **YOUNG, Joshua Abba New York, 10016 (US)**

(74) Representative: **Carpmaels & Ransford LLP One Southampton Row London WC1B 5HA (GB)**

(54) **SYSTEMS AND METHODS FOR IDENTIFICATION AND REFERRAL OF AT-RISK PATIENTS TO EYE CARE PROFESSIONAL**

(57)    A computer-implemented method for identifying one or more patients at risk of having an undetected ophthalmic condition is described. The method may make use of non-ophthalmic data; pre-process the data to generate a culled dataset. The model may be trained and tested based on separate portions of the culled dataset. Finally the model may output, based on the analyzing the data, an indication of the existence or non-existence of one or more ophthalmic conditions.

FIG. 15

**Description**

**BACKGROUND**

Conventional identification and referral of at-risk patients from primary care practitioners (PCPs) to eye care professionals remains problematic. Many people suffer from vision loss as a result of undiagnosed or untreated ophthalmic conditions.

In the United States alone, for example, an estimated 1.9 million people suffer from vision loss as a result of undiagnosed or untreated ophthalmic conditions. For the majority of these, an estimated 1.2 million people, the cause is cataract and vision may be recovered with the appropriate referral to an ophthalmic surgeon. However, for 700,000 Americans, this vision loss is due to undiagnosed or untreated age-related macular degeneration (AMD), glaucoma, or diabetic retinopathy, and vision loss will remain unrecoverable for the majority of these patients. The impact of poor eyesight is manifest in its potentiation of comorbidities, particularly in increasing the risk of disability in patients with cognitive impairment.

Improvements are needed.

**SUMMARY**

The identification and referral of patients at-risk of vision loss from primary care practitioners (PCPs) to eye care professionals remains problematic. A 2010 study identified a number of barriers including a lack of access to ophthalmic screening within the setting of the PCP's office. Some regional efforts have been made to improve the efficiency of triage of patients at risk for glaucoma and diabetic retinopathy; however, existing initiatives triage patients on only a few demographic and co-morbidity parameters, whereas many systemic associations have been identified for AMD, cataract, diabetic retinopathy, glaucoma, and OSD.

Artificial intelligence (AI) modeling techniques are becoming increasingly important in ophthalmology in particular and medicine in general. In ophthalmology, AI is employed to calculate intraocular lens (IOL) powers, predict glaucoma progression, recognize diabetic retinopathy, and classify ocular tumors. To our knowledge, AI has not yet been employed to triage primary care patients for ophthalmology referral. Herein, we report the development, validation, and testing of multiple predictive AI models for five sight-threatening ocular pathologies (*i.e.*, AMD, cataract, diabetic retinopathy, glaucoma, and OSD) that might be employed by PCPs to triage patients for referral to eyecare care professionals.

The present disclosure relates to identification and referral of at-risk patients from primary care practitioners (PCPs) to eye care professionals. As an example, methods described herein may comprise computer-implemented methods for identifying one or more patients at risk of having an undetected ophthalmic condition. A computer or system may receive non-ophthalmic data; pre-process the non-ophthalmic data to generate a culled dataset comprising a subset of the non-ophthalmic data. The AI system or model may be trained, based at least on a first portion of the culled dataset. The model may be tested, based at least on a second portion of the culled dataset different from the first portion. The model may receive non-ophthalmic patient data, analyze that data to determine the existence or non-existence of one or more ophthalmic conditions. The model may output, based on the analyzing the non-ophthalmic patient data, an indication of the existence or non-existence of one or more ophthalmic conditions.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a computer-implemented method for identifying one or more patients at risk of having an undetected ophthalmic condition. The computer - implemented method also includes receiving non-ophthalmic data; pre-processing the non-ophthalmic data to generate a culled dataset may include a subset of the non-ophthalmic data; training, based at least on a first portion of the culled dataset, a model; testing, based at least on a second portion of the culled dataset different from the first portion, the model; receiving non-ophthalmic patient data; analyzing, using the model, the non-ophthalmic patient data to determine the existence or non-existence of one or more ophthalmic conditions; and outputting, based on the analyzing the non-ophthalmic patient data, an indication of the existence or non-existence of one or more ophthalmic conditions. Other embodiments of this aspect include corresponding computer systems, apparatus, and computer programs recorded on one or more computer storage devices, each configured to perform the actions of the methods.

One general aspect includes a digital health tool for identifying patients at higher risk for the presence of ophthalmic pathology. The digital health tool also includes a user interface configured to receive a patient data may include non-ophthalmic data; one or more processors configured to: select a model; analyze, using the model, the non-ophthalmic patient data to determine the existence or non-existence of one or more ophthalmic conditions; and output an indication of the existence or non-existence of one or more ophthalmic conditions. Other embodiments of this aspect include corresponding computer systems, apparatus, and computer programs recorded on one or more computer storage devices, each configured to perform the actions of the methods.

One general aspect includes a computer-implemented method for identifying one or more patients at risk for the presence of ophthalmic pathology. The computer - implemented method also includes selecting a model; analyzing, using the model, non-ophthalmic patient data to determine the existence or non-existence of ophthalmic pathology; and outputting an indication of the existence or non-existence of the ophthalmic pathology. Other embodiments of this aspect include corresponding computer systems, apparatus, and computer programs recorded on one or more computer storage devices, each configured to perform the actions of the methods.

## BRIEF DESCRIPTION OF THE DRAWINGS

The following drawings show generally, by way of example, but not by way of limitation, various examples discussed in the present disclosure. In the drawings:

FIG. 1 depicts the model accuracy by pathology for several machine learning algorithms.
FIG. 2 depicts a box plot of the most important features for exudative AMD
FIG. 3 depicts a box plot of the most important features for non exudative AMD
FIG. 4 depicts a box plot of the most important features for cataracts.
FIG. 5 depicts a box plot of the most important features for OSD.
FIG. 6 depicts a box plot of the most important features for glaucoma.
FIG. 7 depicts a box plot of the most important features for Type 1 PDR.
FIG. 8 depicts a box plot of the most important features for Type 1 NPDR.
FIG. 9 depicts a box plot of the most important features for Type 2 PDR.
FIG. 10 depicts a box plot of the most important features for Type 2 NPDR.
FIG. 11 depicts the receiver operating characteristic (ROC) for exudative AMD
FIG. 12 depicts the ROC for non-exudative AMD
FIG. 13 depicts the ROC for cataracts.
FIG. 14 depicts the ROC for OSD.
FIG. 15 depicts the ROC for glaucoma.
FIG. 16 depicts the ROC for Type 1 PDR.
FIG. 17 depicts the ROC for Type 1 NPDR.
FIG. 18 depicts the ROC for Type 2 PDR.
FIG. 19 depicts the ROC for Type 2 NPDR.
FIG. 20 shows a flow diagram.
FIG. 21 shows a flow diagram.
FIG. 22 shows a flow diagram.
FIG. 23 shows a flow diagram.

## DETAILED DESCRIPTION

The present disclosure relates to a computer-implemented method for identifying one or more patients at risk of having an undetected ophthalmic condition. A computer or system may receive non-ophthalmic data; pre-process the non-ophthalmic data to generate a culled dataset comprising a subset of the non-ophthalmic data. The artificial intelligence (AI) system or model may be trained, based at least on a first portion of the culled dataset. The model may be tested, based at least on a second portion of the culled dataset different from the first portion. The model may receive non-ophthalmic patient data, analyze that data to determine the existence or non-existence of one or more ophthalmic conditions. Finally the model may output, based on the analyzing the non-ophthalmic patient data, an indication of the existence or non-existence of one or more ophthalmic conditions.

The present disclosure relates to a digital health tool for identifying patients at higher risk for the presence of ophthalmic pathology. The digital health tool comprises a user interface configured to receive patient data comprising non-ophthalmic data. It also comprises one or more processes which can select a model, analyze the non-ophthalmic patient data to determine whether the patient is likely to have one or more ophthalmic conditions, and output that analysis.

The present disclosure relates to a computer-implemented method for identifying patients at risk for ophthalmic pathology using non-ophthalmic patient data. The method comprises selecting a model, using the model to analyze non-ophthalmic data to determine whether the patient likely has an ophthalmic pathology, and outputting that result to a user.

Although statistical techniques such as ANOVA can give insight into the relationships among a few clinical parameters, risk stratification and incorporation of multiple demographic, pharmacologic, and comorbidity attributes are well suited to AI modeling. AI is generally divided into two broad categories, though many more than these two exist. Machine learning (ML), including decision tree models, organize parameters (i.e., attributes or features) into strata to predict outcomes. ML is particularly useful for elucidating relationships among clinical parameters. Deep learning (DL) techniques consisting largely of neural networks, including convolutional neural networks (CNNs), recurrent neural networks (RNNs), and perceptrons often improve predictive performance over ML but do so at the cost of opacity and interpretability regarding how their predictions are made.

Multiple artificial intelligence (AI) strategies were built and compared to yield models that may be employed by PCPs to triage patients for referral to eyecare care professionals.

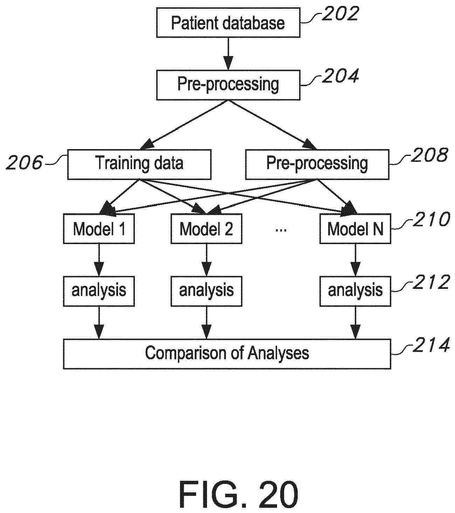

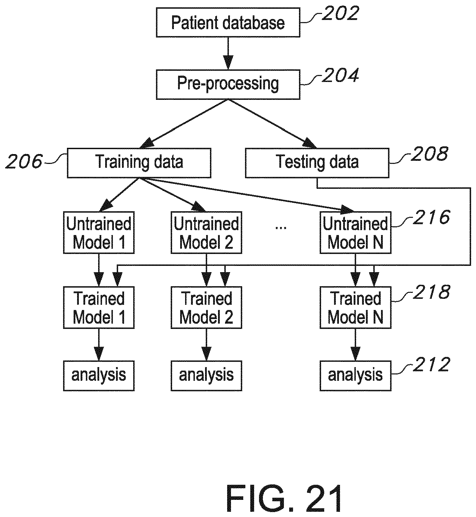

Figure 20 shows a diagram of the method. Data based on one or more subjects may be collected, for example into a database 202. This data may be examined to remove data which is unhelpful or sparse and to limit outliers at the pre-processing step 204. Before identifying a particular model for training and testing, the pre-processed data may be divided into two groups: training data 206 and testing data 208 (also sometimes called the validation data). The first set of data, the training data 206, may be used to train at least one, and possible several models 210. After the models 210 have been trained using the training data 206, they are then tested on the testing data 208. They can output their analysis and the results may be compared at step 214 comparison of analyses. In another depicture, Figure 21 shows how the training data 206 may be fed into the untrained models 216 to create the trained models 218. The testing data 208 may be then fed into the trained models 218 to create the analyses 212 and the prediction or likelihood that the patient has an ophthalmic pathology.

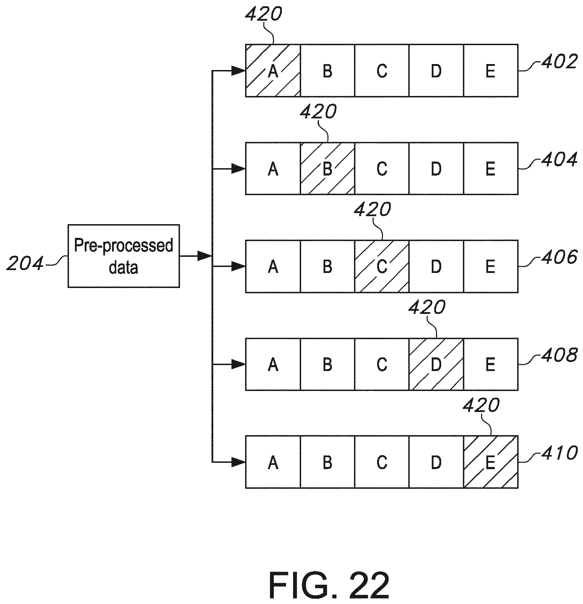

Figure 22 shows an example of cross-validation, which may be a modified form of creating and adjusting the models. In this instance the pre-processed data 204 may be replicated, in this example it may be replicated five times 402 - 410. In each instance of the data replication, the data may be further sub-divided or partitioned into a number of partitions. In this instance the partitions are labelled A, B, C, D, and E. For each replication of the data 402, 404, 406, 408, and 410, the data may be further one partition 420 (shaded to distinguish) may be used as the training data 206 to train the untrained models 216 and create trained models 218. The trained models 218 then use the remaining partitions to test the models. Thus each model may be trained at least once on each partition. This procedure helps to prevent over-fitting of the models to the data.

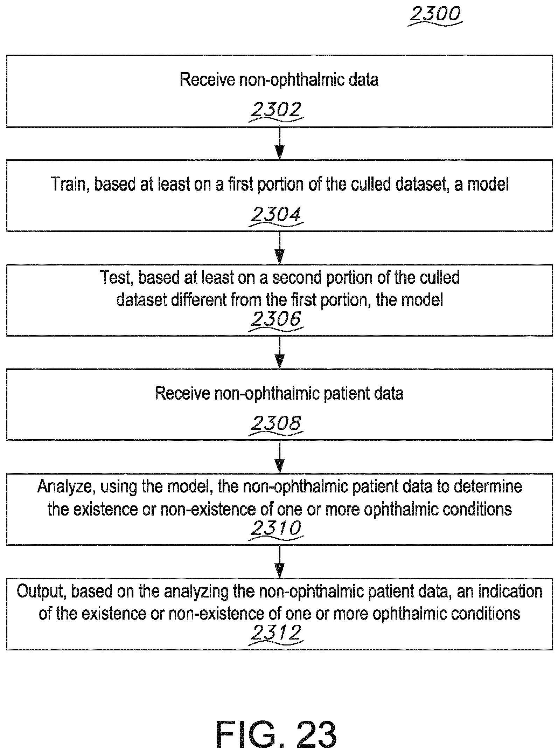

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. Figure 23 illustrates a flow diagram of a computer-implemented method 2300 for identifying one or more patients at risk of having an undetected ophthalmic condition. The computer-implemented method includes receiving non-ophthalmic data, at 2302. The non-ophthalmic data may be based on one or more subjects. As an example, the non-ophthalmic data may be historical patient data collected across a plurality of subjects. The non-ophthalmic data may be pre-processed to generate a culled dataset may include a subset of the non-ophthalmic data. At 2304, the method may comprise training, based at least on a first portion of the culled dataset, a model. At 2306, the method may comprise testing, based at least on a second portion of the culled dataset different from the first portion, the model. At 2308, non-ophthalmic patient data may be received. The non-ophthalmic patient data may be based on a target patient. For example, the non-ophthalmic data used to train or test the model may be based on one or more subjects distinct from or excluding the target patient. As a further example, the model may be trained and tested on data not associated with the target patient. However other data may be used. At 2310, the method may comprise analyzing, using the model, the non-ophthalmic patient data to determine the existence or non-existence of one or more ophthalmic conditions. At 2312, the method may comprise outputting, based on the analyzing the non-ophthalmic patient data, an indication of the existence or non-existence of one or more ophthalmic conditions. Additional data may be used to update the model. For example, the model may be re-trained or re-tested on new data and the updated model may be used in the same or similar manner as described herein. Other embodiments of this aspect include corresponding computer systems, apparatus, and computer programs recorded on one or more computer storage devices, each configured to perform the actions of the methods.

AI techniques have in common the process of "training," the adjustment of importance (i.e., weights) of attributes or intermediate values, based upon a set of data referred to as a training set. The model performance may be then assessed against another set of data called the test set. Similar model performance on training and test sets demonstrates model generalizability. The advent of large clinical databases has made possible the construction and training of both ML and neural network AI models. To this end, we employ a large commercial electronic health record (EHR) database that includes demographic, diagnostic, and therapeutic data to create and curate an ophthalmologically-focused dataset from which predictive models of multiple eye-diseases can be built. We chose to compare several different AI approaches to create models that might be employed by PCPs to triage patients for referral to an eyecare care professional. The models thus created use non-ophthalmic clinical and demographic data to assess relative risk scores for AMD, cataract, OSD, glaucoma, and diabetic retinopathy.

Abbreviations:

AI = artificial intelligence; AMD = age-related macular degeneration; AUC = area under the curve; BMI = body mass index; CNN = convolutional neural network; DL = Deep learning; EHR = electronic health record; EQUALITY = Eye Care Quality and Accessibility Improvement in the Community; GLM = Generalized Linear Model; ICD-10 = International Classification of Diseases, 10th Revision; IOL, intraocular lens; ML = machine learning; NLP = natural language processing; NPDR = nonproliferative diabetic retinopathy; OR = odds ratio; OSD = ocular surface disease; PCPs = primary care practitioners; PDR = proliferative diabetic retinopathy; ROC = receiver operating characteristic; RNN = recurrent neural network; eye care professional = ECP.

**Methods**

Data Source

In one example, a case-controlled study used data from Optum's Pan-Therapeutic EHR database (Optum PanTher EHR). Optum PanTher EHR consists of data primarily from the United States and represents clinical information from more than 80 million patients including at least 7 million patients in each US census region. Data from multiple EHR platforms including Cerner, Epic, GE, and McKesson are analyzed by Optum by means of natural language processing (NLP) to extract information about diagnoses, biometrics, laboratory results, procedures, and medications. Optum PanTher EHR draws upon a network of more than 140,000 providers at more than 700 hospitals and 7,000 clinics.

Outcome Measures

In this example, the method sought to predict the diagnosis of five major eye pathologies: AMD, cataract, diabetic, OSD, glaucoma, and retinopathy. Classification of AMD was based on Optum PanTher EHR International Classification of Diseases, 10th Revision (ICD-10) codes and subdivided into non-exudative (H35.31%) and exudative (H35.32%) groups in which "%" represents a wildcard. Classification of cataract required a more restrictive definition than simply H25%. Since no ICD-10 code distinguishes visually significant cataract from those of lesser impact, we chose to use cataract surgery as a surrogate for visually significant cataract. For this study, cataract was defined by the cataract surgery CPT codes of 66982 or 66984 rather than by ICD-10. Classification of diabetic retinopathy was based on Optum PanTher EHR ICD-10 codes and subdivided into type 1 NPDR (H10.31% - H10.34%), type 1 PDR (H10.35%), type 2 NPDR (H11.31% - HI 1.34%), and type 2 PDR (H11.35%). Glaucoma was defined by the presence of one or more of three criteria: an ICD-10 code of H40.1% (open angle glaucoma), the prescription of glaucoma medication, or the presence of a CPT code indicating glaucoma surgery. This definition was developed to capture not only patients with a recorded diagnosis of glaucoma but also those patients being treated for glaucoma or high-risk ocular hypertension for whom the diagnosis of glaucoma was not recorded in the Optum EHR. Table 1 lists the inclusion criteria for glaucoma. Similar to cataract, OSD required narrower criteria than simply H04.1% and H02.88% since these codes do not distinguish OSD requiring treatment from more mild presentations. For this study, OSD was defined rather restrictively as patients receiving cyclosporine ophthalmic emulsion 0.05%, cyclosporine ophthalmic solution 0.09%, or lifitegrast ophthalmic solution 5%.

**Table 1** Inclusion criteria for glaucoma consisted of prescription of one or more of the listed medications or listed procedures.

| **Beta Blockers** |
|---|
| Levobunolol (Betagan, Akbeta) |
| Carteolol (Ocupress) |
| Metipranolol (Optipranolol) |
| Timolol (Timoptic, Betimal, Istalol) |
| Timolol Gel (Timoptic Xe) |
| Betaxolol (Betoptic, Betoptic S) |
| |
| **Alpha Agonists** |
| Apraclonidine (Iopidine) |

(continued)

| | |
|---|---|
| Brimonidine (Alphagan, Alphagan P) | |
| Dipivefrin (Propine) | |
| | |
| **Carbonic Anhydrase Inhibitors** | |
| Dorzolamide (Trusopt) | |
| Brinzolamide (Azopt) | |
| | |
| **Prostaglandin Analogs** | |
| Latanoprost (Xalatan) | |
| Bimatoprost 0.01% (Lumigan) | |
| Travoprost (Travatan Z) | |
| Tafluprost (Zioptan) | |
| Latanoprostene Bunod (Vyzulta) | |
| | |
| **Rho Khinase Inhibitors** | |
| Netarsudil (Rhopressa) | |
| | |
| **Combined Medications** | |
| Dorzolamide / Timolol (Cosopt And Cospot Pf) | |
| Brimonidine/Timolol (Combigan)). | |
| Brinzolamide/Brimonidine (Simbrinza) | |
| Netarsudil / Latanoprost (Rocklatan) | |
| | |
| **Glaucoma Procedures** | |
| 0191T | Insertion of anterior segment aqueous drainage device, without extraocular reservoir, internal approach, into the trabecular meshwork; initial insertion |
| 0253T | Insertion of anterior segment aqueous drainage device, without extraocular reservoir, internal approach, into the suprachoroidal space |
| 0376T | Insertion of anterior segment aqueous drainage device, without extraocular reservoir, internal approach, into the trabecular meshwork; each additional device insertion (List separately in addition to code for primary procedure) |
| 0449T | Insertion of aqueous drainage device, without extraocular reservoir, internal approach, into the subconjunctival space; initial device |
| 0450T | 0450T Insertion of aqueous drainage device, without extraocular reservoir, internal approach, into the subconjunctival space; each additional device (List separately in addition to code for primary procedure) |
| 0474T | Insertion of anterior segment aqueous drainage device, with creation of intraocular reservoir, internal approach, into the supraciliary space |
| 65820 | Goniotomy |
| 65855 | Trabeculoplasty laser |
| 66174 | Transluminal dilation of aqueous outflow canal; without retention of device or stent |

(continued)

| 66175 | Transluminal dilation of aqueous outflow canal; with retention of device or stent |
|---|---|
| 66179 | Aqueous shunt to extraocular equatorial plate reservoir, external approach; without graft |
| 66180 | Aqueous shunt to extraocular equatorial plate reservoir, external approach; with graft |
| 66183 | Insertion of anterior segment aqueous drainage device, without extraocular reservoir, external approach |
| 66184 | Revision of aqueous shunt to extraocular equatorial plate reservoir; without graft |
| 66185 | Revision of aqueous shunt to extraocular equatorial plate reservoir; with graft |
| 66710 | ciliary body destruction by cyclophotocoagulation, trans-scleral approach |
| 66711 | ciliary body destruction by cyclophotocoagulation, endoscopic approach (ECP) |

Machine Learning (ML)

A number of distinct ML approaches may be taken to model the outcomes described above. In this example the approaches consisted of Generalized Linear Model (GLM), L1-regularized logistic regression, random forest, XGBoost, and J-48 decision trees.

Example Data Pre-Processing

The Optum PanTher EHR data consisted of 380 attributes including demographic information, diagnoses, biometrics, laboratory results, procedures, and medications. Since some of these attributes, particularly some of the laboratory tests, may be only sparsely represented, the data may be pruned to remove attributes (i.e., "features" in ML) with more than 20% missing values. Missing values may be imputed with the median value for continuous variables (e.g., BMI), with a 'Missing' group for categorical variables (e.g., smoke or alcohol usage), and with the most frequent value for binary variables (e.g., levels of lab test results). Winsorization of the data may be performed by replacing values less than the 0.1 percentile value with the 0.1 percentile value and replacing values greater than the 99.9 percentile value with the 99.9 percentile value, respectively. Further feature engineering may be performed to remove or combine highly correlated features such as "Rheumatoid Arthritis / Collagen Vascular Disease" and its highly correlated cognate "Connective Tissue Disease". These feature engineering steps may be performed individually for each case-controlled dataset of each sub-pathology. In this example, the resultant datasets exhibited between 142 and 182 features after the above-described culling. The feature exclusion datasets for each of the nine sub-pathologies in this example were modeled employing each of five distinct modeling strategies to produce a total of 45 individual ML models. Other machine learning models may also be used with this method.

Example Model Strategies

Link 'logit' or logistic regression from the family 'binomial' of GLM may be employed to fit models using maximum likelihood optimization. The dependent variable, or the outcome to be predicted from a given set of independent variables, is binary and therefore logistic regression was chosen. This technique concerns itself with the probability that the dependent variable demonstrates occurrence or non-occurrence of an event; in this case, the recording of a particular diagnosis. It is therefore a classification algorithm. If we assume the probability of occurrence of an event is 'p', where $p \in [0, 1]$, then the probability of non-occurrence of that event will be (1-p).
Logistic regression formula is given as:

$$Y = log\left(\frac{p}{1-p}\right) = \beta_0 + \beta_i X_i$$

Where:

Y is the dependent variable

$X_i$ is an independent variable

$\beta_0$ is the population Y-intercept

$\beta_i$ is the slope value of the line drawn between the dependent and the corresponding independent variable ($X_i$)

It is noteworthy that (p/1-p) is the odds ratio (OR) of occurrence of an event. For an OR value greater than 1, the probability of occurrence is more than 50% and is therefore more likely than the non-occurrence.

Logistic regression, L1-regularized logistic regression, random forest, and XGBoost models may be used, for instance, in python (3.8.5) employing the Scikit-learn (0.23.2) and XGBoost (1.2.0) libraries. In this example, 80% of the data were used for training and 20% of the data were used for testing with 5-fold cross validation (FIG. 22). Grid-search may be employed to optimize hyperparameters. For L1-regularized logistic regression, the regularization strength C may be tuned. In random forest algorithms, the space of the number of trees and the maximum depth of each tree combination may be searched. The hyper-parameter tuning for XGBoost may include the learning rate and the maximum depth of each tree. Machine learning modeling pipeline may be established, and information of missing values fit and learnt from the training data may be applied to the test dataset to avoid information leakage. J48 decision tree modeling, a Java-based implementation of the C4 tree, may be performed in the WEKA ML workbench (University of Waikato, Hamilton, New Zealand). Ten-fold cross validation may be employed with an initial leaf size of 2% of the dataset.

Example Results

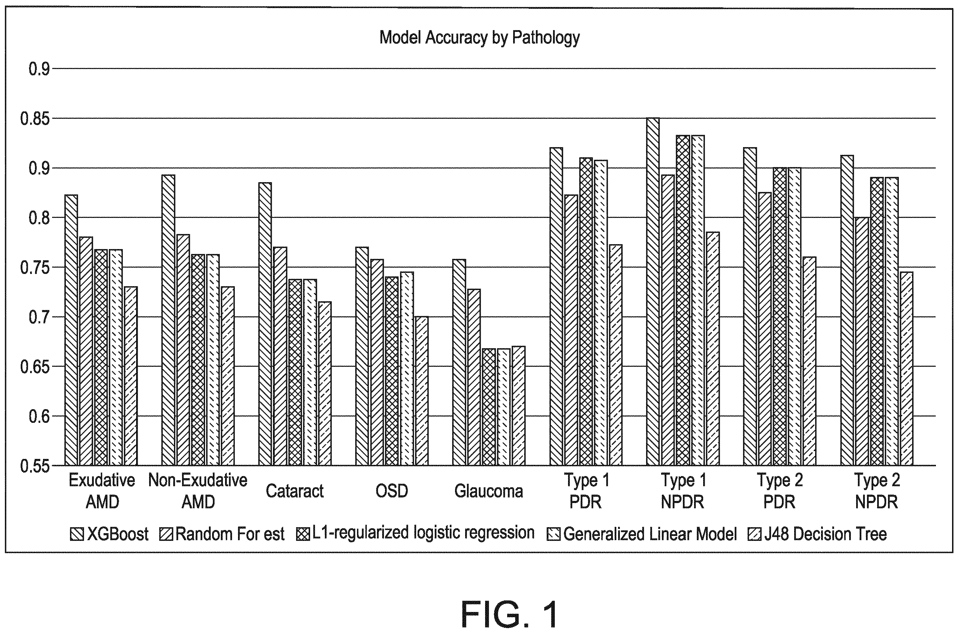

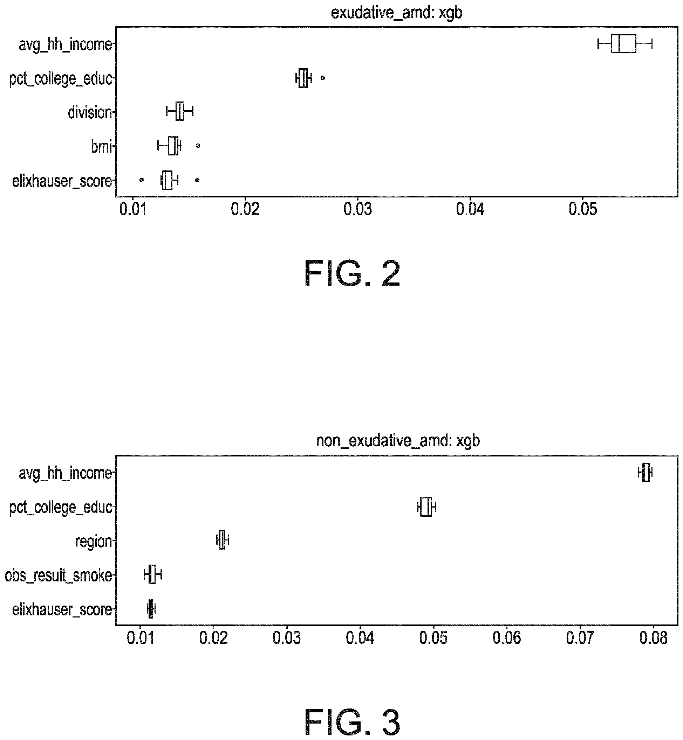

Case control study populations varied by pathology from 395,140 in the case of visually significant cataract to 7,440 in the case of OSD treated with lifitegrast or cyclosporine (Table 2). The performance of different ML strategies varied as well (Figure 1, summary in Table 3 and details in Table 4), but in all cases, XGBoost demonstrated the best performance, showing, respectively, prediction accuracy and AUC of 77.4% and 0.858 for exudative AMD, 79.2% and 0.879 for non-exudative AMD, 78.6% and 0.878 for visually significant cataract, 72.2% and 0.803 for OSD requiring medication, 70.8% and 0.785 for glaucoma, 82.2% and 0.911 for type 1 PDR, 85.0% and 0.924 for type 1 NPDR, 82.1% and 0.900 for type 2 PDR, and 81.3% and 0.891 for type 2 NPDR (Table 4). XGBoost identified a number of clinical attributes that were important to diagnosis prediction (Figure 3).

**Table 2** Case control study populations by pathology

| Case Control Study Populations by Pathology | Population |
|---|---|
| Exudative Age-Related Macular Degeneration | 64,150 |
| Non-Exudative Age-Related Macular Degeneration | 229,678 |
| Cataract Requiring Surgery | 395,140 |
| Glaucoma | 385,514 |
| Ocular Surface Disease Requiring Medication | 7,440 |
| Type I Non-Proliferative Diabetic Retinopathy | 41,308 |
| Type I Proliferative Diabetic Retinopathy | 8,930 |
| Type II Non-Proliferative Diabetic Retinopathy | 311,854 |
| Type II Proliferative Diabetic Retinopathy | 42,064 |

**Table 3** Model Accuracy and Odds Ratio by Pathology

| Pathology | Model Accuracy | Odds Ratio |
|---|---|---|
| Exudative AMD | 77% | 3.39 |
| Non-Exudative AMD | 79% | 3.78 |
| Cataract | 79% | 3.65 |
| Ocular Surface Disease | 77% | 3.41 |
| Glaucoma | 71% | 2.44 |
| Type 1 PDR | 87% | 6.58 |

(continued)

| Pathology | Model Accuracy | Odds Ratio |
|---|---|---|
| Type 1 NPDR | 86% | 6.30 |
| Type 2 PDR | 82% | 4.56 |
| Type 2 NPDR | 81% | 4.21 |

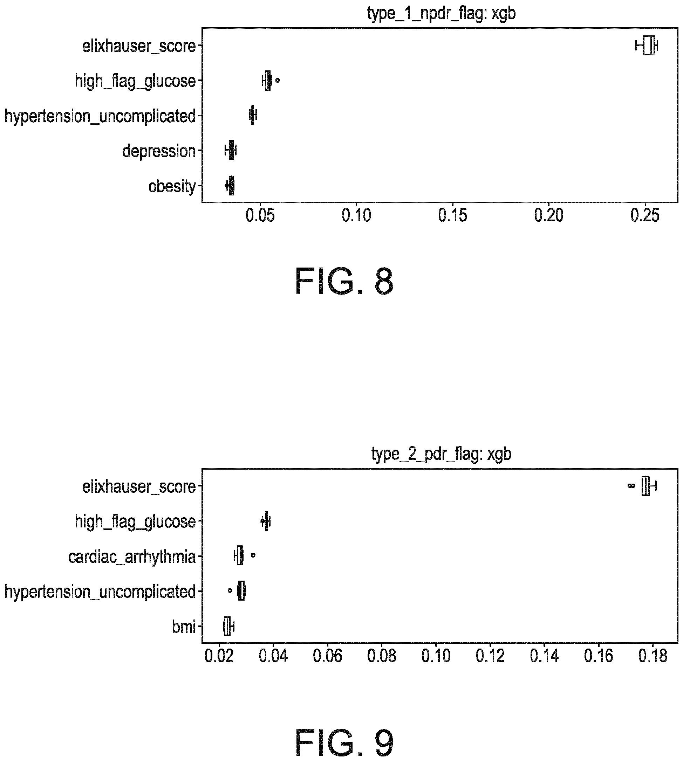

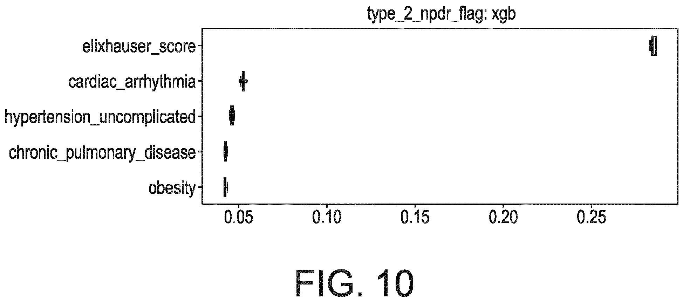

The top-performing models in this example identified the following clinical features that were primarily contributing to the predictions for each pathology are noted here and quantified in box plots in Figures 2-10:

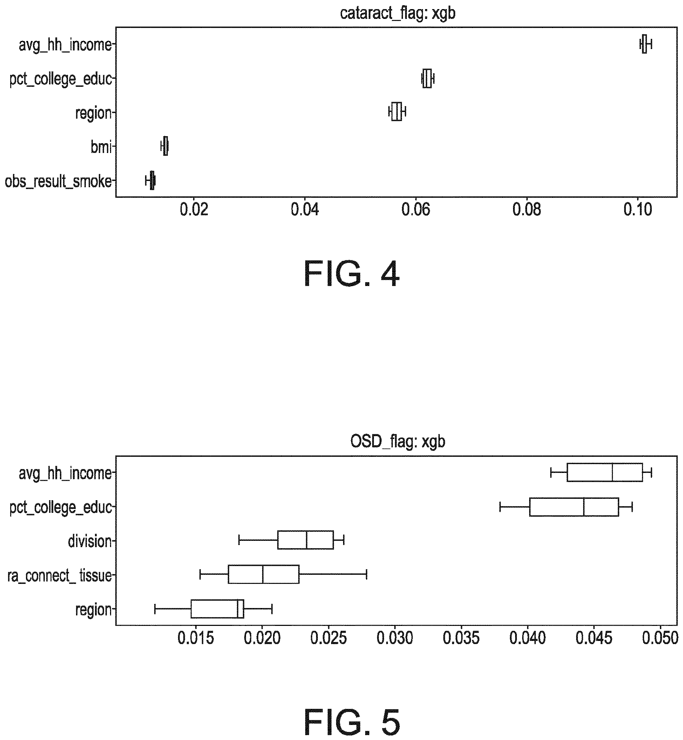

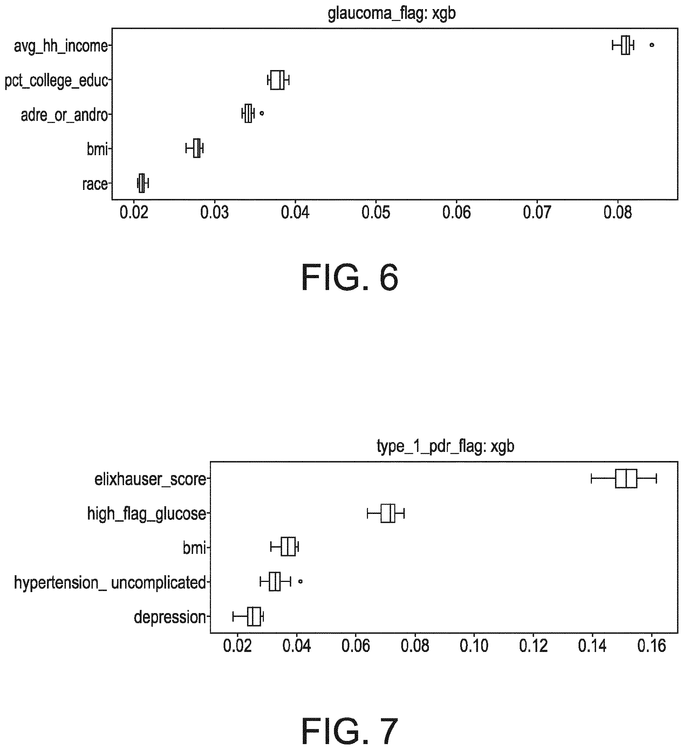

- Exudative AMD diagnosis prediction was associated, in order of importance, with average household income, percent college education, geographical division (Middle Atlantic, East North Central, East South Central, New England, South Atlantic/West South Central, Mountain, West North Central, Pacific, Unknown/Other), body mass index (BMI), and Elixhauser score (comorbidity index). Regional differences across the United States may reflect differences in environment, socioeconomics, quality of healthcare, and other factors that vary between regions and communities, and can therefore be expected to generalize to differences between countries in other continents and between regions in other countries. (FIG. 2)
- Non-exudative AMD demonstrated similar associations. In order of importance, these were average household income, percent college education, region (Northeast, Midwest, South, West, Other/Unknown), smoking, and Elixhauser score. (FIG. 3)
- Cataract clinical associations, in order of importance, included average household income, percent college education, region, BMI, and smoking. (FIG. 4)
- OSD associations included in order of importance, included average household income, percent college education, geographical division, rheumatoid arthritis and connective tissue disease, and region. (FIG. 5)
- Glaucoma clinical associations, in order of importance, included average household income, percent college education, adrenal or androgen use, BMI, and race. (FIG. 6)
- Diabetic retinopathy associations varied over different sub-pathologies (Type 1 PDR, Type 1 NPDR, Type 2 PDR, Type 2 NPDR) but generally included Elixhauser score, high serum glucose, BMI, hypertension, chronic pulmonary disease, depression, cardiac arrhythmia, and obesity. (FIGS 7-10)

The complete results of each XGBoost model for this example, including performance and associations, are shown in Table 4 below.

Table 4 Complete results of various algorithms for various pathologies.

| Pathology | Algorithm | Accuracy (95% CI) | AUC (95% CI) | Sensitivity | Specificity |
|---|---|---|---|---|---|
| Exudative AMD | XGB | 0.774(0.767,0.781) | **0.858(0.851,0.863)** | 0.769 | 0.778 |
| Exudative AMD | RF | 0.73(0.722,0.738) | 0.817(0.81,0.825) | 0.745 | 0.715 |
| Exudative AMD | LR-l1 | 0.718(0.71,0.726) | 0.794(0.786,0.802) | 0.716 | 0.72 |
| Exudative AMD | LR | 0.718(0.71,0.726) | 0.794(0.786,0.801) | 0.717 | 0.72 |
| Exudative AMD | J48 | 0.681 | 0.721 | 0.707 | 0.660 |
| | | | | | |
| Non-Exudative AMD | XGB | 0.792(0.788,0.796) | **0.879(0.876,0.882)** | 0.801 | 0.783 |
| Non-Exudative AMD | RF | 0.733(0.729,0.737) | 0.823(0.82,0.827) | 0.768 | 0.698 |
| Non-Exudative AMD | LR-l1 | 0.713(0.709,0.717) | 0.794(0.79,0.798) | 0.729 | 0.697 |
| Non-Exudative AMD | LR | 0.713(0.709,0.717) | 0.794(0.79,0.798) | 0.727 | 0.7 |
| Non-Exudative AMD | J48 | 0.681 | 0.725 | 0.741 | 0.622 |
| | | | | | |
| Cataract | XGB | 0.786(0.783,0.789) | **0.878(0.875,0.88)** | 0.796 | 0.776 |
| Cataract | RF | 0.721(0.718,0.724) | 0.811(0.808,0.814) | 0.749 | 0.693 |

(continued)

| Pathology | Algorithm | Accuracy (95% CI) | AUC (95% CI) | Sensitivity | Specificity |
|---|---|---|---|---|---|
| Cataract | LR-l1 | 0.689(0.686,0.692) | 0.767(0.764,0.771) | 0.683 | 0.695 |
| Cataract | LR | 0.689(0.686,0.692) | 0.767(0.764,0.771) | 0.683 | 0.695 |
| Cataract | J48 | 0.665 | 0.710 | 0.702 | 0.628 |
| | | | | | |
| OSD | XGB | 0.722(0.699,0.745) | **0.803(0.78,0.824)** | 0.708 | 0.735 |
| OSD | RF | 0.709(0.686,0.732) | 0.771(0.747,0.795) | 0.749 | 0.669 |
| OSD | LR-l1 | 0.69(0.667,0.713) | 0.757(0.732,0.782) | 0.691 | 0.688 |
| OSD | LR | 0.695(0.672,0.718) | 0.757(0.733,0.782) | 0.688 | 0.702 |
| OSD | J48 | 0.651 | 0.702 | 0.675 | 0.628 |
| | | | | | |
| Glaucoma | XGB | 0.708(0.705,0.711) | **0.785(0.782,0.788)** | 0.689 | 0.728 |
| Glaucoma | RF | 0.679(0.676,0.682) | 0.741(0.738,0.745) | 0.656 | 0.702 |
| Glaucoma | LR-l1 | 0.618(0.615,0.621) | 0.669(0.665,0.673) | 0.622 | 0.614 |
| Glaucoma | LR | 0.618(0.615,0.621) | 0.669(0.665,0.673) | 0.619 | 0.617 |
| Glaucoma | J48 | 0.620 | 0.647 | 0.647 | 0.593 |
| | | | | | |
| Type 1 PDR | XGB | 0.822(0.804,0.84) | **0.911(0.897,0.924)** | 0.816 | 0.828 |
| Type 1 PDR | RF | 0.773(0.754,0.792) | 0.861(0.846,0.878) | 0.802 | 0.744 |
| Type 1 PDR | LR-l1 | 0.812(0.794,0.83) | 0.895(0.881,0.91) | 0.847 | 0.777 |
| Type 1 PDR | LR | 0.808(0.79,0.826) | 0.894(0.88,0.91) | 0.829 | 0.787 |
| Type 1 PDR | J48 | 0.724 | 0.804 | 0.761 | 0.686 |
| | | | | | |
| Type 1 NPDR | XGB | 0.850(0.842,0.858) | **0.924(0.919,0.93)** | 0.85 | 0.85 |
| Type 1 NPDR | RF | 0.795(0.786,0.804) | 0.872(0.864,0.879) | 0.799 | 0.79 |
| Type 1 NPDR | LR-l1 | 0.835(0.827,0.843) | 0.908(0.902,0.915) | 0.847 | 0.824 |
| Type 1 NPDR | LR | 0.835(0.827,0.843) | 0.908(0.902,0.915) | 0.847 | 0.824 |
| Type 1 NPDR | J48 | 0.738 | 0.796 | 0.756 | 0.721 |
| | | | | | |
| Type 2 PDR | XGB | 0.821(0.813,0.829) | **0.900(0.893,0.907)** | 0.841 | 0.801 |
| Type 2 PDR | RF | 0.777(0.768,0.786) | 0.858(0.85,0.865) | 0.763 | 0.79 |
| Type 2 PDR | LR-l1 | 0.799(0.79,0.808) | 0.880(0.873,0.887) | 0.834 | 0.763 |
| Type 2 PDR | LR | 0.8(0.791,0.809) | 0.880(0.873,0.887) | 0.847 | 0.753 |
| Type 2 PDR | J48 | 0.711 | 0.774 | 0.674 | 0.748 |
| | | | | | |
| Type 2 NPDR | XGB | 0.813(0.81,0.816) | **0.891(0.888,0.893)** | 0.845 | 0.782 |
| Type 2 NPDR | RF | 0.751(0.748,0.754) | 0.833(0.83,0.836) | 0.751 | 0.752 |
| Type 2 NPDR | LR-l1 | 0.791(0.788,0.794) | 0.866(0.863,0.869) | 0.843 | 0.739 |
| Type 2 NPDR | LR | 0.791(0.788,0.794) | 0.866(0.863,0.869) | 0.844 | 0.739 |

(continued)

| Pathology | Algorithm | Accuracy (95% CI) | AUC (95% CI) | Sensitivity | Specificity |
|---|---|---|---|---|---|
| Type 2 NPDR | J48 | 0.696 | 0.742 | 0.635 | 0.757 |
| | | | | | |
| XGB: XGBoost | | | | | |
| RF: Random Forest | | | | | |
| LR-l1: l1-regularized logistic regression | | | | | |
| LR: Logistic Regression without regularization | | | | | |
| J48: Decision tree | | | | | |

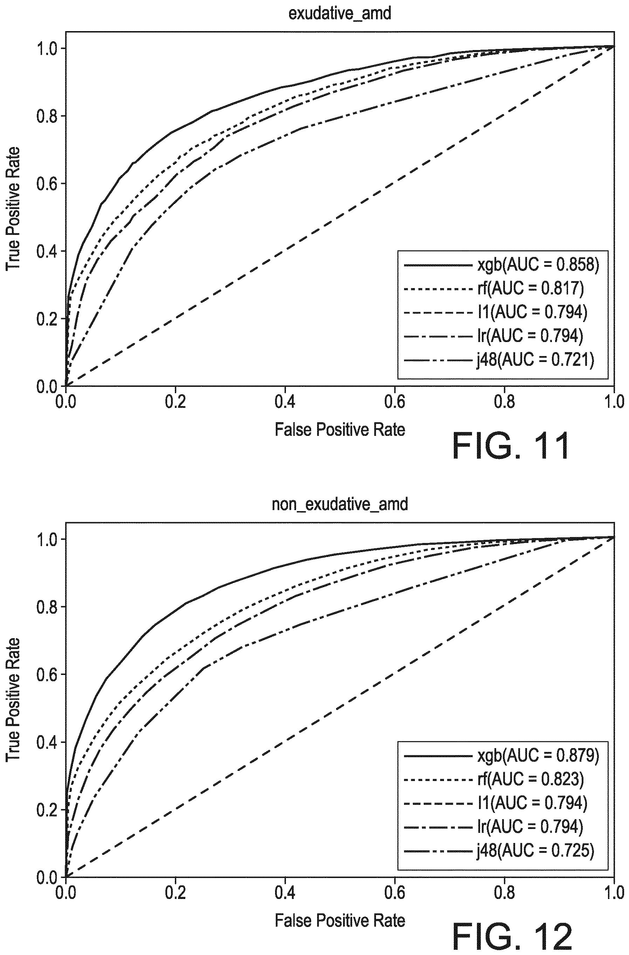

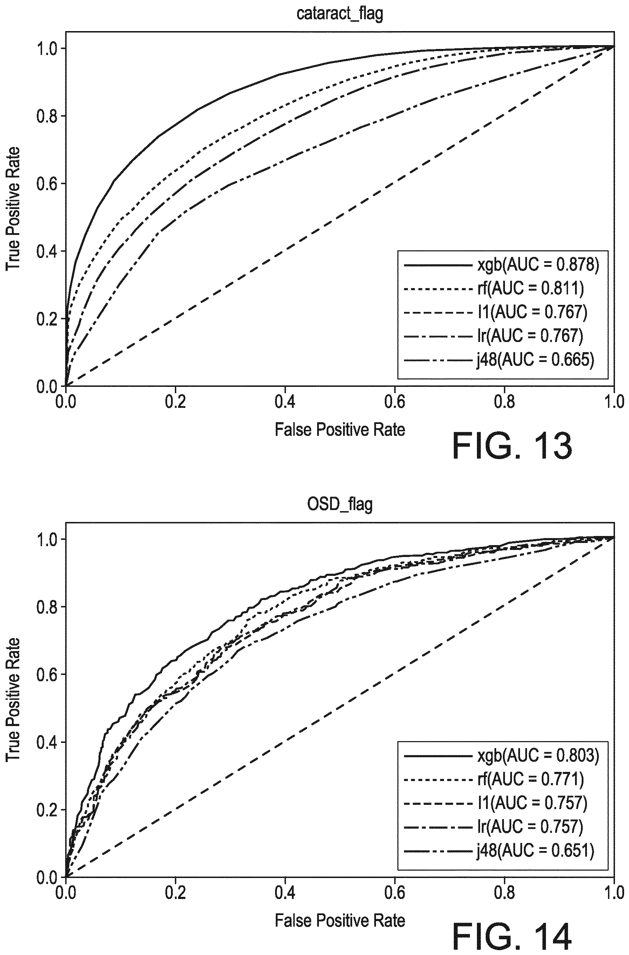

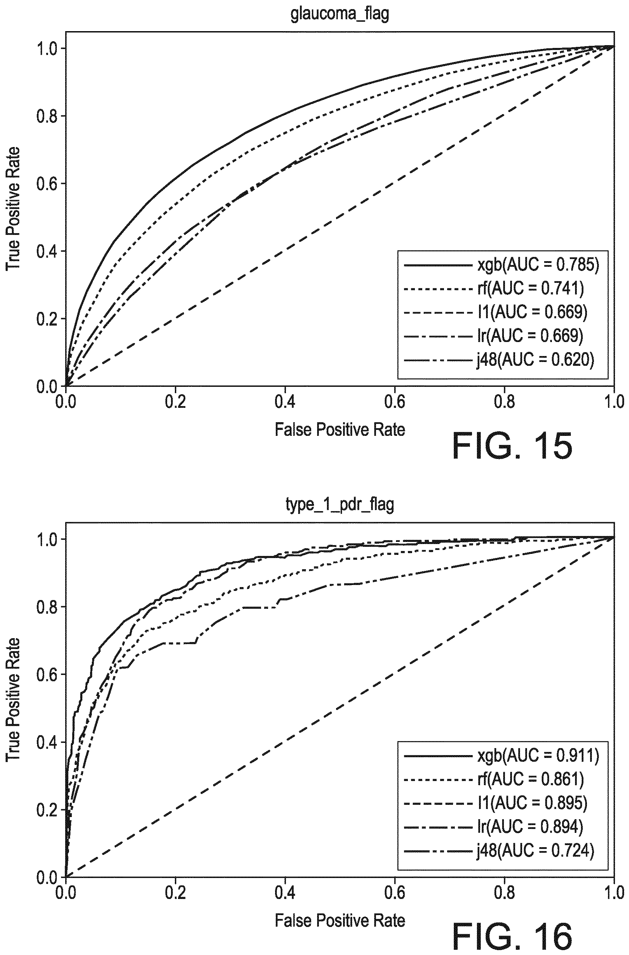

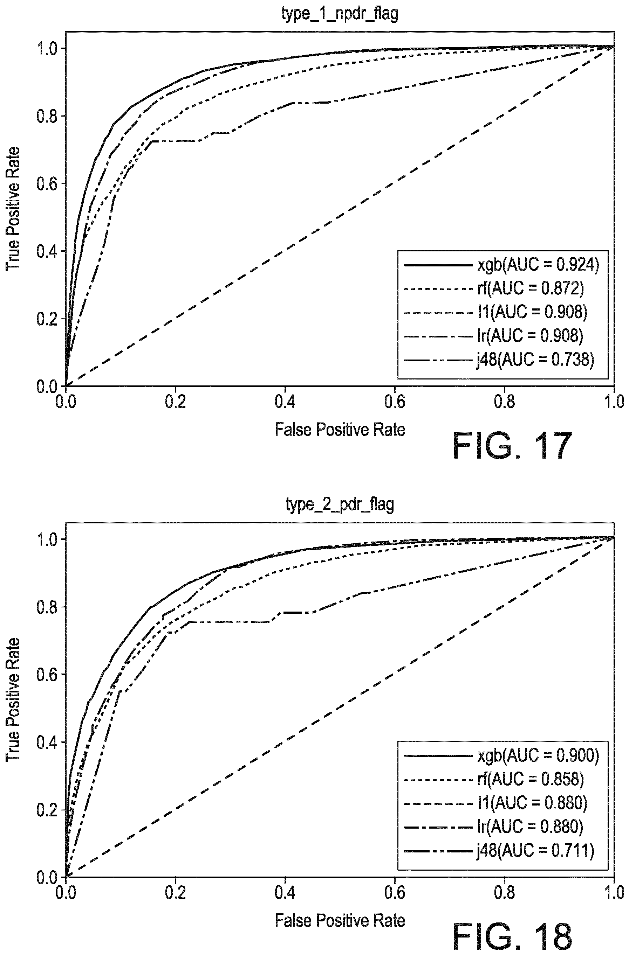

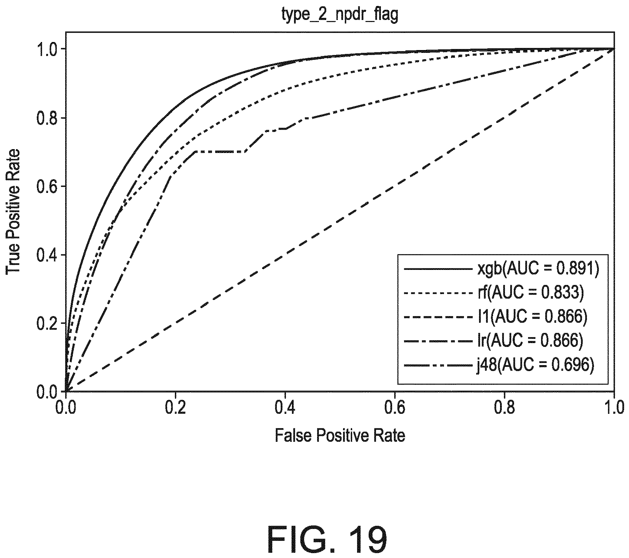

Details of the AUC for this example are shown in the associated ROC curves displayed in Figures 11-19, one for each pathology.

**Discussion**

Performance of the Example Models

Beginning with EHR data of more than 80 million patients, the final study populations totaled 1,486,078 patients, 50% of whom were controls. In addition to the enormous patient population, this example demonstrated 90 different AI models for five major pathologies and nine sub-pathologies in order to arrive at the most predictive model for each pathology.

The goal of this effort is to create a digital health tool to identify patients at higher risk for the presence of ophthalmic pathology and to do this based solely on the sort of non-ophthalmic data to which a PCP would have access. This digital health tool does not propose to either make definitive ophthalmic diagnoses nor to predict development of future pathology. Rather, the digital health tool seeks to identify patients whose clinical and demographic context is associated with the presence of AMD, cataract, clinically significant diabetic retinopathy, glaucoma, or OSD disease of a magnitude requiring pharmacological therapy.

Performance in predicting the presence of pathology for this example ranged between 71% in the case of glaucoma to 87% in the case of type 1 proliferative diabetic retinopathy, with an average performance of 80% across all groups. Since the intent is to identify at-risk patients, these performance values may be used to determine disease odds ratios according to the method described by Hogue, Gaylor, and Schulz, for example, as described in Altman, Douglas G., Practical Statistics for Medical Research. Chapman & Hall (1991). Since the case control study populations for each pathology were evenly split between pathology and control, random selection of a patient would yield a 50% chance of pathology. If a model performs at 80% accuracy, it is, in essence, identifying a population for whom there is an 80% risk of pathology. The calculation of odds ratio ($\theta$) is:

$$\theta = \frac{P_M/P_O}{(1 - P_M)/(1 - P_O)}$$

In which $\theta$ = odds ratio, $P_M$ = fraction of model predicted population with pathology, $P_O$ = fraction of original population with pathology. Since the original study population (Po) was case-controlled with 50% pathology, both Po and (1- Po) = 0.5 and the formula simplifies to:

$$\theta = \frac{P_M}{1 - P_M}$$

Applying this to each of the models provides a clinically useful measure. The models in this example identify patients with elevated odds ratios of prevalence of pathology of between 2.44 in the case of glaucoma to 6.58 in the case of type 1 proliferative diabetic retinopathy with an average odds ratio of approximately 4 as shown in Table 3. Application of such a model in the clinical setting could allow a PCP to identify patients nearly four times more likely to have ophthalmic pathology. Such a tool would bring a substantial benefit in the triage and referral of at-risk patients to eye care professionals.

## Example Data and Outcome Engineering

The data used to produce and test these models in this example were obtained from the Optum Pan-Therapeutic EHR database (Optum PanTher EHR), though other databases could be used. These data consist of diagnostic and procedure codes, biometric data such as BMI and vital signs, demographic information including socioeconomic and geographical information, laboratory results, and medications prescribed. This information does not include the physician notes that might provide rationale for the diagnoses recorded. Indeed, since only a limited number of diagnoses may be listed on a claim, it is possible that some extant diagnoses may have gone unrecorded. On the other hand, diagnoses like cataract and OSD may be over-represented since the ICD-10 taxonomy does not distinguish between clinically significant cataract and OSD from cases in which these pathologies were subclinical. Indeed, it would be of little clinical utility to build an AI model that detects subclinical cataracts.

This example demonstrates the challenge of identifying clinically relevant diagnoses from large datasets. A 2018 study in JAMA Ophthalmology investigated the precision of ICD-10 codes for patients with uveitis and found that 13 of 27 uveitides were imprecisely defined and that multiple codes were employed to describe the same pathology. A 2020 study of ocular pathology in stroke patients noted fewer patients with glaucoma than anticipated and attributed this to the lack of ophthalmology clinic data. Patients may be on glaucoma medications without a concurrent ICD code recorded for glaucoma, suggesting that a diagnosis of glaucoma may have been recorded in the patient's medical record before incorporation into the dataset. Therefore to defining the glaucoma cohort in this example, was expanded to include those patients who met one or more of three criteria: an ICD-10 code of H40.1% (open angle glaucoma), the prescription of glaucoma medication, or the presence of a CPT code indicating glaucoma surgery (see Table 1). This definition was developed in order to both detect glaucoma patients without glaucoma ICD-10 codes and to exclude patients inappropriately labeled as glaucoma by ICD-10. This definition resulting in a substantial winnowing of the glaucoma cohort from 1,368,700 (50% of whom were controls) to 385,514 patients. Similar data pre-processing may be required of other databases to include all patients who might be at risk.

A similar approach may be taken to the cataract and OSD study populations. Cataract and OSD are among the most frequently recorded diagnoses on claims. Cataract, in particular, is nearly ubiquitous in elderly patients and was the most common ophthalmic ICD-10 diagnosis of those examined in this example. Since only a subset of these require cataract surgery, the detection of cataract alone is not clinically useful. ICD-10 coding does not distinguish between cataracts requiring surgery and those that do not. However, CPT coding, in a sense does make this distinction. Therefore, we chose a CPT of 66984 (cataract extraction with intraocular lens) and 66982 (complex cataract extraction) as the criteria for clinically significant cataracts. This narrowing of inclusion criteria reduced the cataract study population from 2,087,836 (50% of whom were controls) to 395,140 patients in this example. OSD coding is even more problematic. A large number of ICD-10 codes are available and clinical significance is difficult to establish. The initial cohort of OSD patients and controls for this database totaled 1,182,912 patients. In order to model the clinical context associated with OSD, a a restrictive criterion was chosen: the prescription of topical cyclosporine or lifitegrast. This greatly reduced the OSD population to only 7,440 patients, but these represent patients with clinically meaningful disease. No outcome engineering measures were applied to the AMD groups or to the diabetic retinopathy groups, each of which was defined by its corresponding ICD-10 code.

## An Example of Clinical Attributes and Feature Engineering

The initial dataset in this example included a large number of attributes or "features" (in the language of ML), totaling 380 individual parameters. In order to produce models that would not be burdensome for the clinician to employ, the number of attributes required by each model was reduced. This reduction and modification of model parameters is referred to as "feature engineering". In order for a feature to be included in the final model, several criteria needed to be met. The feature must play a significant role in the model's outcome. It is self-evident that features that do not contribute substantially to a model may be discarded with little impact on model performance. In the case of the XGBoost models, parameter optimization was performed by grid search algorithm. The second feature inclusion criterion was non-correlation with other features. In some cases, such as between weight and BMI, the correlation is evident. However, the correlation between other clinical features only becomes clear on analysis. The issue of feature correlation highlights a difference between AI and traditional risk-analysis studies. When studied individually, certain attributes such as obesity and socioeconomic status may be identified as disease risk factors. However, when viewed collectively, the importance of one of these may be reduced if the two attributes are highly correlated. The third criterion for feature inclusion was high frequency in the dataset. Some of the laboratory values, particularly serum fibrinogen, were so sparse in this particular dataset that exclusion of the feature was preferable to the alternatives of sample reduction or interpolation. Two thresholds for feature sparsity were used in this example. Models were built upon datasets that excluded features with more than 20% missing values. Feature engineering substantially benefits from guidance by clinical domain experts and our feature and outcome engineering was clinically informed, particularly in the realm of the diagnostic criteria described above.

Utilization Data and Generalizability

The data in the example given above do not contain the richness of a complete medical record. It is therefore impossible to establish the criteria under which the clinicians made the diagnoses recorded, hence the outcome engineering maneuvers to establish stricter criteria, e.g., using CPT codes for cataract surgery to identify clinically significant cataract patients. At the same time, models built upon these sorts of data are more generalizable and available than models built upon more specific and perhaps more idiosyncratic data sources. These are precisely the sorts of data available to PCPs, making these models more easily deployable than models built upon a specific medical record system. Indeed, the availability of these data is illustrated by the example noted above which included more than 80 million patients from disparate healthcare systems.

Hierarchical Relationships

It should be noted that the clinical features identified as relevant by each of the pathology models should be viewed as correlative but not necessarily causative. It is better to think of the collection of clinical values as a patient's clinical milieu rather than as a collection of individual risk factors. While it is difficult to imagine that college education is itself a risk factor for pathology, its correlation and importance to a given model should not be discounted since it contributed to the model's predictiveness of the presence of pathology in the example described above.

All of this is not to say that causation may not exist in the relation between some of these features and the pathologies modeled. Highly multi-dimensional clinical AI studies like the example above may identify previously unrecognized factors that directly influence pathogenesis. However, causative connection cannot be established by these sorts of study and would require a more traditional experimental approach. Although the J-48 decision tree models did not perform as well as the GLM or XGBoost strategies in the example case, they are informative in that they describe hierarchical relationships among clinical features. As an example, the J-48 model for glaucoma identifies race, systemic steroids, and anti-diabetic medication use as important clinical features. However, the model dictated the order in which these factors should be considered, assessing race only after it is established whether the patient takes anti-diabetic medications, and assessing systemic steroid use only after these first two attributes have been determined. Such a hierarchical relationship among clinical features may be enormously difficult to establish in traditional reduced-dimensional scientific queries. This gestalt approach to multi-dimensional clinical context is one of the strengths of this method.

Prediction

The purpose of these models is prediction. However, in order for the work to be properly applied, a clear understanding of "prediction" must first be established. These models predict the presence of extant pathology. They should be of value in the identification of populations in which these pathologies are substantially more prevalent than in the general population. The models should not be employed to make a diagnosis for an individual patient, but rather to identify patients at risk of having undetected AMD, cataract, diabetic retinopathy, glaucoma, or OSD. Further, these models are built upon clinical data in which an ophthalmic pathology is or is not present. That is to say, these models are not constructed to predict the development of future pathology. It may or may not be the case that a particular clinical context, as defined by the multi-dimensional features incorporated into the models, may predict the development of future disease. It would be inappropriate to employ these models as a pure diagnosis. These models predict the presence of ophthalmic pathology based upon non-ophthalmic data and may be best used for triage and referrals from non-ophthalmologists to eye care care professionals. Other uses are contemplated.

The present disclosure comprises at least the following aspects:

Aspect 1. A computer-implemented method for identifying one or more patients at risk of having an undetected ophthalmic condition, the method comprising: receiving non-ophthalmic data; pre-processing the non-ophthalmic data to generate a culled dataset comprising a subset of the non-ophthalmic data; training, based at least on a first portion of the culled dataset, a model; testing, based at least on a second portion of the culled dataset different from the first portion, the model; receiving non-ophthalmic patient data; analyzing, using the model, the non-ophthalmic patient data to determine the existence or non-existence of one or more ophthalmic conditions; and outputting, based on the analyzing the non-ophthalmic patient data, an indication of the existence or non-existence of one or more ophthalmic conditions.

Aspect 2. The method of Aspect 1, wherein the non-ophthalmic patient data is based on a target patient, and wherein the non-ophthalmic data is based on one or more subjects distinct from the target patient. The non-ophthalmic data may be based on one or more subjects excluding the target patient.

Aspect 3. The method of Aspect 1, wherein the one or more ophthalmic conditions comprises age-related macular degeneration (AMD), cataract, diabetic retinopathy, glaucoma, or ocular surface disease (OSD).

Aspect 4. The method of Aspect 1, wherein the pre-processing comprises feature engineering.

Aspect 5. The method of Aspect 4, wherein the feature engineering comprises removing or combining highly correlated features.

Aspect 6. The method of Aspect 1, wherein the pre-processing comprises the removing of one or more attributes with more than 20% missing values.

Aspect 7. The method of Aspect 1, wherein the pre-processing comprises replacing values less than the 0.1 percentile value with the 0.1 percentile value and replacing values greater than the 99.9 percentile value with the 99.9 percentile value.

Aspect 8. The method of Aspect 1, wherein the model is based on at least a logistic regression model.

Aspect 9. The method of Aspect 1, wherein the model is based on at least the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of the line drawn between the dependent and the corresponding independent variable ($X_i$).

Aspect 10. A digital health tool for identifying patients at higher risk for the presence of ophthalmic pathology, the digital health tool comprising: a user interface configured to receive a patient data comprising non-ophthalmic data; one or more processors configured to: select a model; analyze, using the model, the non-ophthalmic patient data to determine the existence or non-existence of one or more ophthalmic conditions; and output an indication of the existence or non-existence of one or more ophthalmic conditions.

Aspect 11. The digital health tool of Aspect 10, wherein the one or more ophthalmic conditions comprises age-related macular degeneration (AMD), cataract, diabetic retinopathy, glaucoma, or ocular surface disease (OSD).

Aspect 12. The digital health tool of Aspect 10, wherein the model is based on at least a logistic regression model.

Aspect 13. The digital health tool of Aspect 10, wherein the model is based on at least on the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of the line drawn between the dependent and the corresponding independent variable ($X_i$).

Aspect 14. A computer-implemented method for identifying one or more patients at risk for the presence of ophthalmic pathology, the method comprising: selecting a model; analyzing, using the model, non-ophthalmic patient data to determine the existence or non-existence of ophthalmic pathology; and outputting an indication of the existence or non-existence of the ophthalmic pathology.

Aspect 15. The method of claim 14, wherein the non-ophthalmic patient data is based on a target patient, and wherein the model is based on non-ophthalmic data associated with one or more subjects distinct from the target patient. The non-ophthalmic data may be based on one or more subjects excluding the target patient.

Aspect 16. The method of Aspect 14, wherein the ophthalmic pathology comprises age-related macular degeneration (AMD), cataract, diabetic retinopathy, glaucoma, or ocular surface disease (OSD).

Aspect 17. The method of Aspect 14, wherein the ophthalmic pathology comprises one or more variables of the non-ophthalmic data that correlate to a risk of age-related macular degeneration (AMD), cataract, diabetic retinopathy, glaucoma, or ocular surface disease (OSD).

Aspect 18. The method of Aspect 14, further comprising pre-processing the non-ophthalmic patient data.

Aspect 19. The method of Aspect 18, wherein the pre-processing comprises feature engineering.

Aspect 20. The method of Aspect 19, wherein the feature engineering comprises removing or combining highly correlated features.

Aspect 21. The method of Aspect 18, wherein the pre-processing comprises the removing of one or more attributes with more than 20% missing values.

Aspect 22. The method of Aspect 18, wherein the pre-processing comprises replacing values less than the 0.1 percentile value with the 0.1 percentile value and replacing values greater than the 99.9 percentile value with the 99.9 percentile value.

Aspect 23. The method of Aspect 14, wherein the model is based on at least a logistic regression model.

Aspect 24. The method of Aspect 14, wherein the model is based on at least on the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of the line drawn between the dependent and the corresponding independent variable ($X_i$).

Although shown and described is what is believed to be the most practical and preferred embodiments, it is apparent that departures from specific designs and methods described and shown will suggest themselves to those skilled in the art and may be used without departing from the spirit and scope of the invention. For example, the systems, devices and methods described herein prediction of ophthalmic diagnoses are based on non- ophthalmic data. It will be appreciated by the skilled artisan that the devices and methods described herein may not be limited to this area and could be used for other diagnostic areas. The present invention is not restricted to the particular constructions described and illustrated, but should be constructed to cohere with all modifications that may fall within the scope of the appended claims.

**Claims**

1. A computer-implemented method for identifying one or more patients at risk of having an undetected ophthalmic condition, the method comprising:

   receiving non-ophthalmic data;
   pre-processing the non-ophthalmic data to generate a culled dataset comprising a subset of the non-ophthalmic data;
   training, based at least on a first portion of the culled dataset, a model;
   testing, based at least on a second portion of the culled dataset different from the first portion, the model;
   receiving non-ophthalmic patient data;
   analyzing, using the model, the non-ophthalmic patient data to determine the existence or non-existence of one or more ophthalmic conditions; and
   outputting, based on the analyzing the non-ophthalmic patient data, an indication of the existence or non-existence of one or more ophthalmic conditions.

2. The method of claim 1, wherein the non-ophthalmic patient data is based on a target patient, and wherein the non-ophthalmic data is based on one or more subjects distinct from the target patient.

3. The method of claim 1, wherein the one or more ophthalmic conditions comprises age-related macular degeneration (AMD), cataract, diabetic retinopathy, glaucoma, or ocular surface disease (OSD).

4. The method of claim 1, wherein the pre-processing comprises feature engineering.

5. The method of claim 4, wherein the feature engineering comprises removing or combining highly correlated features.

6. The method of claim 1, wherein the pre-processing comprises removing of one or more attributes with more than 20% missing values.

7. The method of claim 1, wherein the pre-processing comprises replacing values less than the 0.1 percentile value with the 0.1 percentile value and replacing values greater than the 99.9 percentile value with the 99.9 percentile value.

8. The method of claim 1, wherein the model is based on at least a logistic regression model.

9. The method of claim 1, wherein the model is based on at least the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of a line drawn between the dependent and the corresponding independent variable ($X_i$).

10. A digital health tool for identifying patients at higher risk for the presence of ophthalmic pathology, the digital health tool comprising:

a user interface configured to receive a patient data comprising non-ophthalmic data;
one or more processors configured to:

select a model;
analyze, using the model, the non-ophthalmic patient data to determine the existence or non-existence of one or more ophthalmic conditions; and
output an indication of the existence or non-existence of one or more ophthalmic conditions.

11. The digital health tool of claim 10, wherein the one or more ophthalmic conditions comprises age-related macular degeneration (AMD), cataract, diabetic retinopathy, glaucoma, or ocular surface disease (OSD).

12. The digital health tool of claim 10, wherein the model is based on at least a logistic regression model.

13. The digital health tool of claim 10, wherein the model is based on at least on the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of a line drawn between the dependent and the corresponding independent variable ($X_i$).

14. A computer-implemented method for identifying one or more patients at risk for the presence of ophthalmic pathology, the method comprising:

selecting a model;
analyzing, using the model, non-ophthalmic patient data to determine the existence or non-existence of ophthalmic pathology; and

outputting an indication of the existence or non-existence of the ophthalmic pathology.

15. The method of claim 14, wherein the non-ophthalmic patient data is based on a target patient, and wherein the model is based on non-ophthalmic data associated with one or more subjects distinct from the target patient.

16. The method of claim 14, wherein the ophthalmic pathology comprises age-related macular degeneration (AMD), cataract, diabetic retinopathy, glaucoma, or ocular surface disease (OSD).

17. The method of claim 14, wherein the ophthalmic pathology comprises one or more variables of the non-ophthalmic data that correlate to a risk of age-related macular degeneration (AMD), cataract, diabetic retinopathy, glaucoma, or ocular surface disease (OSD).

18. The method of claim 14, further comprising pre-processing the non-ophthalmic patient data.

19. The method of claim 18, wherein the pre-processing comprises feature engineering.

20. The method of claim 19, wherein the feature engineering comprises removing or combining highly correlated features.

21. The method of claim 18, wherein the pre-processing comprises removing of one or more attributes with more than 20% missing values.

22. The method of claim 18, wherein the pre-processing comprises replacing values less than the 0.1 percentile value with the 0.1 percentile value and replacing values greater than the 99.9 percentile value with the 99.9 percentile value.

23. The method of claim 14, wherein the model is based on at least a logistic regression model.

24. The method of claim 14, wherein the model is based on at least on the logistic regression formula:

$$Y = log\left(\frac{p}{1-p}\right) = \beta_0 + \beta_i X_i$$

Where:

Y is the dependent variable
$X_i$ is an independent variable
$\beta_0$ is population Y-intercept
$\beta i$ slope value of a line drawn between the dependent and the corresponding independent variable ($X_i$).

FIG. 1

FIG. 2

FIG. 3

FIG. 4

FIG. 5

FIG. 6

FIG. 7

FIG. 8

FIG. 9

type_2_npdr_flag: xgb

FIG. 10

exudative_amd

| | |
|---|---|
| —— | xgb(AUC = 0.858) |
| ······ | rf(AUC = 0.817) |
| – – – | l1(AUC = 0.794) |
| – · – · | lr(AUC = 0.794) |
| – ·· – ·· | j48(AUC = 0.721) |

True Positive Rate

False Positive Rate

## FIG. 11

non_exudative_amd

| | |
|---|---|
| —— | xgb(AUC = 0.879) |
| ······ | rf(AUC = 0.823) |
| – – – | l1(AUC = 0.794) |
| – · – · | lr(AUC = 0.794) |
| – ·· – ·· | j48(AUC = 0.725) |

True Positive Rate

False Positive Rate

## FIG. 12

FIG. 13

FIG. 14

glaucoma_flag

FIG. 15

type_1_pdr_flag

FIG. 16

type_1_npdr_flag

FIG. 17

type_2_pdr_flag

FIG. 18

FIG. 19

FIG. 20

FIG. 21

FIG. 22

*2300*

| Receive non-ophthalmic data |
| *2302* |

↓

| Train, based at least on a first portion of the culled dataset, a model |
| *2304* |

↓

| Test, based at least on a second portion of the culled dataset different from the first portion, the model |
| *2306* |

↓

| Receive non-ophthalmic patient data |
| *2308* |

↓

| Analyze, using the model, the non-ophthalmic patient data to determine the existence or non-existence of one or more ophthalmic conditions |
| *2310* |

↓

| Output, based on the analyzing the non-ophthalmic patient data, an indication of the existence or non-existence of one or more ophthalmic conditions |
| *2312* |

# FIG. 23

**European Patent Office**
Europäisches Patentamt
European Patent Office
Office européen des brevets

# EUROPEAN SEARCH REPORT

**Application Number**

EP 22 18 2009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 722 180 B2 (AI TECH INC [KY]; UNIV CALIFORNIA [US]) 28 July 2020 (2020-07-28) * columns 52,53; figures 1, 2b * | 1-24 | INV.<br>G16H10/60<br>G16H50/20<br>G16H50/30<br>G16H50/70 |

**TECHNICAL FIELDS SEARCHED (IPC)**

G16H

The present search report has been drawn up for all claims

| Place of search | Date of completion of the search | Examiner |
|---|---|---|
| Munich | 18 November 2022 | Flores Sanchez, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 2009

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2022

| Patent document cited in search report | | Publication date | Patent family member(s) | | Publication date |
|---|---|---|---|---|---|
| US 10722180 | B2 | 28-07-2020 | AU | 2018347610 A1 | 07-05-2020 |
| | | | CA | 3083035 A1 | 18-04-2019 |
| | | | CN | 112513999 A | 16-03-2021 |
| | | | EP | 3695416 A1 | 19-08-2020 |
| | | | JP | 2021507428 A | 22-02-2021 |
| | | | SG | 11202003337V A | 28-05-2020 |
| | | | US | 2019110753 A1 | 18-04-2019 |
| | | | WO | 2019075410 A1 | 18-04-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALTMAN, DOUGLAS G.** Practical Statistics for Medical Research. Chapman & Hall, 1991